# EUROPEAN PATENT SPECIFICATION

(11) **EP 4 255 516 B1**
(45) Date of publication and mention of the grant of the patent: **13.08.2025**
(21) Application number: 21824305.3
(22) Date of filing: 29.11.2021
(51) Int. Cl.: A61L 26/00, A61K 31/19, A61K 31/327, A61P 17/02

(54) **FORMULATIONS FOR HEALING OF TRAUMATIC LESIONS OF THE SKIN AND MUCOUS MEMBRANES**
FORMULIERUNGEN ZUR HEILUNG TRAUMATISCHER LÄSIONEN DER HAUT UND SCHLEIMHÄUTE
FORMULATIONS POUR LA CICATRISATION DE LÉSIONS TRAUMATIQUES DE LA PEAU ET DE MUQUEUSES

(30) Priority: 01.12.2020 US 202063119722 P
(43) Date of publication of application: 11.10.2023
(73) Proprietor: Castellana, Rossana, 34129 Trieste (IT); Piani, Francesco, 34125 Trieste (IT)
(72) Inventor: Castellana, Rossana, 34129 Trieste (IT); Piani, Francesco, 34125 Trieste (IT)
(74) Representative: Bianchetti & Minoja with Trevisan & Cuonzo IPS SRL
(86) International application number: PCT/EP2021/083345
(87) International publication number: WO 2022/117499

(56) References cited:
- EP-A2- 1 979 053
- WO-A2-2009/152374
- GPG GROUP: "Vi presentiamo la nuova sede GPQ", WIPO, 1 January 2020 (2020-01-01), XP093258752
- ANONYMOUS: "No-Needle SkinBooster", SKIN AESTHETICS, 1 January 2020 (2020-01-01), XP093258772
- ROY ET AL: "Dermal Wound Healing Is Subject to Redox Control", MOLECULAR THERAPY, ELSEVIER INC, US, vol. 13, no. 1, 1 January 2006 (2006-01-01), pages 211 - 220, XP005197711, ISSN: 1525-0016, DOI: 10.1016/J.YMTHE.2005.07.684

## Description

### Technical field

The invention relates to formulations in the form of a cream, an ointment, a liquid, or a gel for use in repairing surgical wounds of skin and mucous membranes. The synergic action of the components of the formulations allows a surprising healing activity of lesions following an odontostomatological intervention like dental extraction, apicectomies, sinus lift, implantology, gingivectomy, periodontal surgery and in the dermatological treatment of slowly healing wounds, spontaneous ulcerations, sores of various origins and necrotic sores including, pyogenic granulomas, chalazion, epulis, fistulas and abscesses.

### Background of the invention

Disruption of the integrity of skin and mucosal surfaces results in the formation of a wound. Wound healing is a complex biological process which restore the tissue integrity. Physiologically, wound healing can be schematically divided into four distinct phases: haemostasis, inflammation, proliferation and tissue remodelling. Immediately after an injury, the haemostasis process begins: the bleeding is controlled by the aggregation of platelets and the subsequent formation of the fibrin clot stops the bleeding and provides a scaffold for the attachment and proliferation of the cells. After 2-3 days, the inflammatory process progresses into the proliferative phase and fibroblasts are attracted into the wound to synthesize granulation tissue. This granulation tissue allows leukocytes to enter the wound site and once the wound is closed, the immature scar can move on to the final remodelling phase which can last up to a year. The main function of fibroblasts in tissue regeneration is to maintain the physical integrity of connective tissue by producing and remodelling the extracellular matrix. Regardless of the aetiology of the wound, whether acute or chronic, the above described repair processes are similar but while acute wounds, e.g. a surgical incision, usually pass through these phases relatively quickly, wounds undergoing delayed healing up to 12 weeks after the initial insult, often as a result of prolonged pathological inflammation, are defined chronic wounds..

Several insults like biofilm formation, microbial invasion, repeated trauma, ischemia, oedema, venous hypertension and effect of mechanical forces, including pressure, may cause healing impairment. These insults lead to poor migration of keratinocytes and the epithelization is also delayed. In these indolent cases, wound bed preparation remains the standard of therapy.

An effective wound bed preparation may involve enzymatic (J. Ramundo et al., J Wound Ostomy Continence Nurs Actions. May-Jun 2008;35(3):273-80; McCarty MS; Adv Wound Care (New Rochelle). 2013 Oct; 2(8): 438-447) and surgical debridement, resident fibroblast stimulation and stimulation of growth factor release (L. Maddaluno et al., Development (2017) 144, 4047-4060), addition of extraneous growth factors to the wound (M. Yadav et al. Indian Journal of Fundamental and Applied Life Sciences (2012), 2 (2) April-June,164 -172), deployment of bioengineered extracellular matrix (F. Urciolo et al., J. Clin. Med. 2019, 8, 2083), collagen and alginates (B. A. Aderibigbe et al., Pharmaceutics, 2018, 10(2), 42), cultured keratinocyte suspension and even bioengineered dermal preparation (G. Schultz. Wound Repair and Regeneration, April 2003, 11 Suppl 1(s1):S1-28) in any combination and also the stem cell therapy holds promise (N. Kosaric et at., Expert Opinion on Biological Therapy, 19 (6), 2019).

The known treatment options remain only moderately effective and often fail to promote the closure of non-healing wounds in susceptible populations, such as aging and diabetic patients or represent a limited remedy in case of wounds by insect or spider bites (Hindawi Publishing Corporation, Case Reports in Emergency Medicine, Volume 2016, Article ID 7640789). Moreover, some of these approaches, like for example the bioengineered skin substitutes, although representing a valid alternative to autografting, induce skin cells in repairing the wound rather than guiding a regeneration process.

Analogously, the surgical wounding of oral mucosa after surgical incision requires, without complications, long recovery times. In order to restore the original physiology of the oral cavity, up to five weeks are usually required but with some possible complications that could delay the complete healing of the wound. In particular, the normal healing response to tooth extraction results in a significant loss of bone and collapse of the surrounding gingiva. In normal healing, a substantial percentage of extraction sites suffers from postoperative complications.

In fact, after tooth extraction blood clots fill the socket and, after one week, the clots are replaced by granulation tissue; after 3 weeks, the granulation tissue is replaced by collagen, and bone formation begins at the base and the periphery of the extraction socket. At 5 weeks, it is estimated that on average two-thirds of the extraction socket is filled with bone. Epithelium was found to require a minimum of three weeks to completely cover the extraction socket, with some extraction sites requiring up to five weeks to completely cover the socket (Amler MH et al. J Am Dent Assoc. 1960;61(7), 32-44; Amler MH Oral Surg Oral Med Oral Pathol, 1969, 27 (3), 309-318). Post-operative complications of the above-mentioned normal healing in response to tooth extraction may include dry socket, dysesthesia, severe infection, pain, swelling, trismus and haemorrhage. All these possible complications significantly increase the total healing time from a surgical incision of the oral mucosa.

Trichloroacetic acid alone or in association with other compounds may be used as "chemical peel" (Monheit GD; J. Dermatol. Surg. Oncol. (1989) 15 (9), 945-950). A 50% trichloroacetic (TCA) solution is commonly used as medium-depth chemoexfoliation which is a frequently used to treat fine rhytides, actinic photodamage, hyperpigmentation, and even actinic-related premalignant changes, such as actinic keratoses.

Chloroacetic acid has been disclosed for the treatment of chronic wounds together with an extracellular polymeric substance solvating system surfactant and buffering agent (WO2009152374). Formulations containing trichloroacetic acid and hydrogen peroxide in a pH range of 2.3-2.6 were also proposed for the treatment of pathologies of the skin and mucosa membranes, such as acne, damage caused by the sun and sun freckles (EP1979053) with some limitations due to the fact that hydrogen peroxide, known as mild antiseptic, may be used on the skin to prevent infection of minor cuts, scrapes, and burns but it is endowed with potential negative effects on tissue repairs and should not be used to treat deep wounds, animal, insect or spider bites, or serious burns (Agency for Toxic Substances and Disease Registry, Buford Hwy NE Atlanta-Hydrogen Peroxide).

More recently, the biological effect of hydrogen peroxide during the wound-healing process was reconsidered: in relatively high concentrations (*i.e.* 3%), hydrogen peroxide displays its strong ability of oxidization and pro-inflammation to disinfect wound tissue (Roy S, et al. Mol Ther 2006; 13 :211-220); however, in lower concentrations (i.e. 1%) it shows good antimicrobial effect and skin tolerability (Capizzi R. et al. Br J Dermatol 2004;151:481-484).On the contrary, an oxidized form of trichloroacetic acid, namely trichloroperoxyacetic acid has never been considered for human use even if *in vitro* tests confirmed disinfectants properties of diluted aqueous solutions against *Bacillus subtilis, Escherichia coli and Staphylococcus aureus* (Sita, F. et al. Journal of Hygiene, Epidemiology, Microbiology and Immunology (1968), 12(3), 370-3).

There is thus a need for innovative pharmacological treatments of surgical wounds on skin and mucous membrane, avoiding the above mentioned post-operative complications and allowing an optimal wound bed preparation.

### Summary of the invention

In order to solve the above-mentioned problems, the present invention provides formulations for repairing skin and mucous membrane wounds.

The wound healing compositions of the invention comprise:
- a first component selected from trichloroacetic in a concentration by weight ranging from 0.08 to 38 %;
- a second component selected from 29-31% aqueous hydrogen peroxide in a concentration by weight ranging from 0.01 to 5 % or trichloroperoxyacetic acid:
   - the relative molar ratio between trichloroacetic acid and hydrogen peroxide is from 4.34 to 4.74; or
   - the relative molecular ratio between trichloroperoxyacetic acid and trichloroacetic acid is from 3.6 to 4.0; said compositions having a pH value from 2.6 to 3.6

The components of these formulations display a synergic action in the treatment of wounds of skin and mucous membranes.

The formulations of the invention are devoid of the potential negative effects of hydrogen peroxide on tissue.

The formulations of the invention are applied topically in the form of cream, ointment, liquid, gel or similar administration forms. The formulations have a rapid effect, quickly reduce swelling and pain, promote wound healing and reduce the times of recovery of chronic wounds, like chronic ulcers bedsores. The use of the formulations is also effective in treating post-operative complications of normal healing in response to tooth extraction including dry socket, dysesthesia, severe infection, pain, swelling, trismus, haemorrhage and in the treatment of slowly healing wounds, spontaneous ulcerations, sores of various origins and necrotic sores including, pyogenic granulomas, chalazion, epulis, fistulas and abscesses.

### Detailed description of the invention

The formulations of the invention are characterized by a concentration of hydrogen peroxide aqueous solution (0.01 to 5 % by weight of a 29-31%, hydrogen peroxide aqueous solution) remarkably lower than that disclosed in the prior art, particularly in EP 1979053 (50-80% by weight).

The concentration of trichloroacetic preferably ranges from 0.1 to 35 % by weight, more preferably from 0.15 to 15 % by weight.

The formulations of the invention may be diluted using a 0,9% w/v aqueous NaCl solution. The diluted formulations secure the viability of the cells of the mucosal membrane and of the skin and are useful to accelerate the repair of the surgical wounds, post traumatic wounds, unhealed wounds, ulcers and bedsores. The pH value of the formulations is higher than 2,6, typically in the range from 2.6 to 3.6.

The liquid formulations of the invention may contain water in a range from 38 to 99% by weight, EDTA disodium salt in a range from 0.001 to 0.5% by weight, glycerol in a range from 0,025 to 15% by weight, aqueous ammonia (28-32%) in a range from 0,03 to 14% by weight, a thickening and texture agent in a range from 0,005 to 5% by weight and optionally 0,8-0,9% w/v of sodium chloride. Preferred thickening and texture agents include hydroxyethylcellulose, guar gum, locust bean gum, xanthan gum, gelatine and hydroxyethyl acrylate/sodium acryloyldimethyl taurate copolymers. More preferred thickening and texture agents comprise hydroxyethyl acrylate/sodium acryloyldimethyl taurate copolymer (Sepineo^{™} d.e.r.m).

The undiluted ointment formulations of the invention may also contain water in a range between 5 and 12% by weight, sodium laurylsulfate in a range from 0.5 to 1.5% in weight, propylene glycol in a range from 10 to 15% by weight, stearyl alcohol in a range between 20 and 30% by weight, white petrolatum in a range between 20 and 30% by weight, aqueous ammonia (28-32%) in a range between 4 and 6% by weight and optionally one or more preservatives in a concentration between 0.02 and 0.05%. Preferred preservatives include propylparaben, methylparaben, sodium benzoate and ethylhexylglycerin. Optionally, the formulation does not contain any preservative. Trichloroacetic acid and trichloroperoxyacetic acid can be alternatively used instead of trichloroacetic acid and hydrogen peroxide. These formulations can optionally be administered after dilution with an isotonic 0,9% w/v NaCl aqueous solution.

The undiluted cream formulations of the invention may contain, in addition to the combination of trichloroacetic acid and hydrogen peroxide or of trichloroperoxyacetic acid and trichloroacetic acid in the proportions above defined, water in a range between 20 and 25% by weight, liquid paraffin in a range between 5 and 12% by weight, propylene glycol in a range from 3 to 7% by weight, glycerine in a range from 8 to 12% by weight, aqueous ammonia 28-32% in a range from 10 to 14% by weight and polysorbate 60 in a range from 1 to 2% by weight. These formulations can optionally be administered after dilution with an isotonic 0,9% w/v NaCl aqueous solution.

The undiluted gel formulations of the invention may contain ethanol in a range between 25 and 35% by weight, propylene glycol in a range between 11 and 15% by weight, aqueous ammonia (28-32% w/v) in a range of 10-14%, diethylene glycol monoethyl ether in a range from 2 to 5% by weight and myristyl alcohol in a range between 1 and 4%. These formulations can optionally be administered after dilution with an isotonic 0,9% w/v NaCl aqueous solution.

### Experimental section

### Example 1

An ointment for repairing skin and mucous membranes wounds has the following composition:

| **Components** | **% (w/w)** |
|---|---|
| Purified water | 11.59 |
| Propylparaben | 0.02 |
| Sodium Laurylsulfate | 1.05 |
| Propylene Glycol | 12.63 |
| Stearyl Alcohol | 26.32 |
| Methylparaben | 0.03 |
| Trichloroacetic acid | 14.60 |
| Hydrogen peroxide 29-31% | 2.21 |
| Aqueous ammonia 28-32% | 5.22 |
| White petrolatum | 26.32 |

This concentrated ointment formulation can be diluted using a 0,9% w/v NaCl aqueous solution to obtain a final liquid formulation with a pH value ≥ 2,6.

### Example 2

A liquid for repairing wounds of skin and mucous membranes has the following composition (Formulation 14-03-01: 1/400 dilution)

| | **Concentrated liquid solution** | **1/10 dilution** | **1/100 dilution** | **1/200 dilution** | **1/400 dilution** |
|---|---|---|---|---|---|
| **Components** | **% (w/w)** | **% (w/w)** | **% (w/w)** | **% (w/w)** | **% (w/w)** |
| Purified water | 38.0 | 93.8 | 99.380 | 99.690 | 99.850 |
| Disodium EDTA | 0.2 | 0.02 | 0.002 | 0.001 | 0.001 |
| Trichloroacetic acid | 33.0 | 3.3 | 0.330 | 0.165 | 0.083 |
| Glycerol | 10.0 | 1.0 | 0.100 | 0.050 | 0.025 |
| Hydrogen peroxide 29-31% | 5.0 | 0.5 | 0.050 | 0.025 | 0.013 |
| Aqueous ammonia 28-32% | 11.8 | 1.2 | 0.118 | 0.059 | 0.030 |
| Sepineo^{™} d.e.r.m. | 2.0 | 0.2 | 0.020 | 0.010 | 0.005 |

The concentrated liquid solution can be diluted up to 1:400 using a 0,9% w/v NaCl aqueous solution to obtain a final liquid formulation with a pH value ≥ 2.6.

### Example 2a

A liquid for repairing skin and mucous membranes wounds has the following composition:

| **Components** | **% (w/w)** |
|---|---|
| Purified water | 41.5 |
| Disodium EDTA | 0.2 |
| Trichloroperoxyacetic acid | 28.3 |
| Trichloroacetic acid | 7 |
| Glycerol | 10 |
| Aqueous ammonia 28-32% | 11.8 |
| Sepineo^{™} d.e.r.m. | 2 |

This concentrated liquid solution can be diluted using a 0,9% w/v NaCl aqueous solution to obtain a final liquid formulation with a pH value ≥ 2,6. Alternatively, trichloroperoxyacetic acid can be prepared *in situ* by addition of equimolar amounts of hydrogen peroxide to trichloroacetic acid.

### Example 3

A cream for repairing skin and mucous membranes wounds has the following composition

| **Components** | **% (w/w)** |
|---|---|
| Purified water | 23.9 |
| Liquid paraffine | 10.0 |
| Trichloroacetic acid | 33.0 |
| Propyleneglycol | 5.0 |
| Hydrogen peroxide 29-31% | 5.0 |
| Aqueous ammonia 28-32% | 11.8 |
| Glycerine | 10.0 |
| Polysorbate 60 | 1.3 |

This concentrated formulation can be diluted using a 0,9% w/v NaCl aqueous solution to obtain a final liquid formulation with a pH value ≥ 2,6.

### Example 4

A gel for repairing skin and mucous membranes wounds has the following composition:

| **Components** | **% (w/w)** |
|---|---|
| Ethanol | 30.8 |
| Trichloroacetic acid | 33.0 |
| Propylene glycol | 13.9 |
| Hydrogen peroxide 29-31% | 5.0 |
| Aqueous ammonia 28-32% | 11.8 |
| Diethylene glycol monoethyl ether | 3.5 |
| Myristyl alcohol | 2.1 |

This concentrated formulation can be diluted using a 0,9% w/v NaCl aqueous solution to obtain a final liquid formulation with a pH value ≥ 2,6.

### Comparative example 1

The only difference from Example 2 (Formulation 14-03-01) is that the components do not contain hydrogen peroxide.

| **Formulation 14-04-03** | |
|---|---|
| **Components** | **% (w/w)** |
| Trichloroacetic acid | 33.0 |
| Glycerol | 10.0 |
| Purified water | 43.0 |
| Aqueous ammonia 28-32% | 11.8 |
| Sepineo ^{™} d.e.r.m. | 2.0 |
| Disodium EDTA | 0.2 |

This concentrated formulation was diluted 1:400, using a 0.9% w/v NaCl solution to obtain a final liquid formulation with a pH value ≥ 2.6.

### Comparative example 2

| **Formulation 14-04-05** | |
|---|---|
| **Components** | **% (w/w)** |
| Glycerol | 10.0 |
| Hydrogen peroxide 29-31% | 5.0 |
| Purified water | 84.6 |
| Aqueous ammonia 28-32% | 0 |
| Sepineo TM d.e.r.m. | 0.2 |
| Disodium EDTA | 0.2 |

This concentrated liquid solution was diluted 1:400, using a 0.9% w/v aqueous NaCl solution to obtain a final liquid formulation with a pH value ≥ 2.6.

The liquid formulations 14-03-01 (TCA plus H₂O₂), 14-04-03 (TCA only) and 14-04-05 (H₂O₂ only) were then biologically evaluated in *in vivo* and *in vitro* tests. These tests confirmed the synergic action of trichloroacetic acid and hydrogen peroxide in accelerating the healing of wounds and ulcers of mucous membranes and skin, even after dilution up to 400 time with a 0,9% NaCl aqueous solution and at a pH value ≥ 2.6.

### Experimental in vitro and in vivo tests

### Experimental Protocol

This study was divided in two steps: in the first, the effectiveness of solutions was verified on an in vitro cell model using NHEK cells (normal human epidermal cell); in the second the effectiveness of solutions was carried out on a mouse animal model mimicking a slow-healing human wound. The solutions tested in both steps included the following combinations:
- 14-03-01 (TCA plus H₂O₂)
- 14-04-03 (TCA only)
- 14-04-05 (H₂O₂ only)
and their effects were compared to an untreated control sample.

In an *in vitro* model, NHEK cells were used to test the efficacy of the solutions in a dose-response study to exclude any cytotoxic effects analyzing cell viability by MTT (3-(4,5-dimethylthiazol-2-yl)-2,5-diphenyl tetrazolium bromide) test. Since the combinations of substances have never been described in the literature, the range tested was based on TCA concentration reported in literature [Yang H, et al., Toxicol In Vitro. 2011 Dec;25(8):1638-43.]. In particular, 1: 100 (20mM); 1: 200 (10mM); 1: 400 (5mM) and 1: 800 (2.5mM) diluted with sterile physiological solution (0.9% NaCl) were tested. 3 different stimulation protocols were selected, as described below:
- 1 treatment only that is left up to max 6 days (total 1 stimulus), protocol A
- 1 treatment / day that is carried out max 6 days (total of 5 stimuli), protocol B
- 1 treatment / alternate days carried out max 6 days (total 3 stimuli), protocol C.

Since the data highlighted the minimum effective dose and the importance of administering an adequate dose, in the subsequent experiments only the 5mM concentration was tested using only the protocols A and C. Subsequently, tests were carried out to evaluate cell proliferation (Crystal Violet assay), migration (wound assay) and production of reactive oxygen species (ROS).

Migration and re-epithelization were investigated in an *in vivo* model using male C57BL/6JOlaHsd mice, excluding any toxic effects induced by the substances tested in protocol A and C. Before administration, the mice were depilated on the right side of the back spine. The depilated area was about 2×2cm. The bedsore model was squeezed in the depilated area by intermittent mechanical extrusion to simulate the natural formation process of bedsores.

In particular,
- 14-03-01 (TCA plus H₂O₂)
- 14-04-03 (TCA only)
- 14-04-05 (H₂O₂ only)

were used at 1: 400 dilutions prepared in sterile physiological solution (0.9% NaCl) to treat skin wounds. At the end of each treatment, a planimetric analysis of the wounds was performed and wound areas were collected to make histological analysis as E/E (hematoxylin/eosin) and Masson staining.

### Methods: in vitro model

### Cell culture

Normal human epidermal keratinocyte cells (NHEK) purchased from Lonza (Basel, Switzerland) were cultured in EpiLife medium (Gibco, Thermo Fisher Scientific, Waltham, MA, USA) containing 60 µM Ca2+ and HKGS (Gibco, Thermo Fisher Scientific) in a 5% CO₂ incubator (Thermo Fisher Scientific) at 37 °C. The medium was replaced every two days, and the cells were used at 70% to 80% confluence [Jia, T et al., Molecules 2019, 24, 3156]. Experiments were conducted at passages 3-6.

### Cell viability

After each stimulation, the NHEK cells were washed with sterile PBS 1× and incubated with DMEM (Dulbecco s modified eagle medium) without red phenol and FBS (fetal bovine serum) containing 1% MTT dye (MTT-Based In Vitro Toxicology Assay Kit; Sigma-Aldrich) for 2 h at 37°C and 5% CO₂ [Uberti, F et al., J Cardiovasc Pharmacol 57: 246-258.]. Cell viability was determined by measuring absorbance using a spectrometer (VICTORX4 multi-label plate reader) at 570 nm with correction at 690 nm and calculated by comparing the results to control cells (100% viable).

### Crystal violet

After each treatment the cells were fixed with 1% glutaraldehyde (Sigma-Aldrich) for 15 min at room temperature, washed, and stained with 100 µL 0.1% aqueous crystal violet (Sigma- Aldrich) for 20 min at room temperature. 100µL microliters of 10% acetic acid were added to multi-well plates and mixed before reading the absorbance at 595 nm using a spectrometer (VICTORX4 multi-label plate reader). The estimated number of cells was calculated by comparing the results to the control cells (control T0), examined on the first treatment, and the variation of the untreated cells was also reported [Uberti F, Cells Tissues Organs. 2017;203(4):215-230. Epub 2016 Nov 25.].

### Wound assay in vitro

A scratch wound healing assay was performed as previously described [Uberti F, et al., Cells Tissues Organs. 2017;203(4):215- 230. Epub 2016 Nov 25.] in confluent monolayer cells using a sterile p200 pipette tip. Afterwards, the cells were stimulated with different preparations in different protocols (A and C) and monitored for 6 days. After each time point, repopulation of the wounded areas was observed under a phase contrast microscope (Leitz, Germany). Using the ImageJ image-processing program, the size of the denuded area was determined at each time point from digital images taken at 6 different areas. The results are expressed as means ± SD (%) of migrated cells.

### ROS production

The rate of superoxide anion release was measured using a standard protocol based on reduction of cytochrome C. In both treated and untreated cells, 100 µL cytochrome C were added and in another sample 100 µL superoxide dismutase were also added for 30 min in an incubator (all substances were from Sigma-Aldrich). The absorbance in culture supernatants was measured at 550 nm using a spectrometer (VICTORX4 multilabel plate reader) and O2 was expressed as the mean ± SD (%) of nanomoles per reduced cytochrome C per microgram of protein compared to the control [Uberti F, et al., Cells Tissues Organs. 2017;203(4):215-230. Epub 2016 Nov 25].

### Results of the in vitro tests

### Dose-response study of cell viability on NHEK cells.

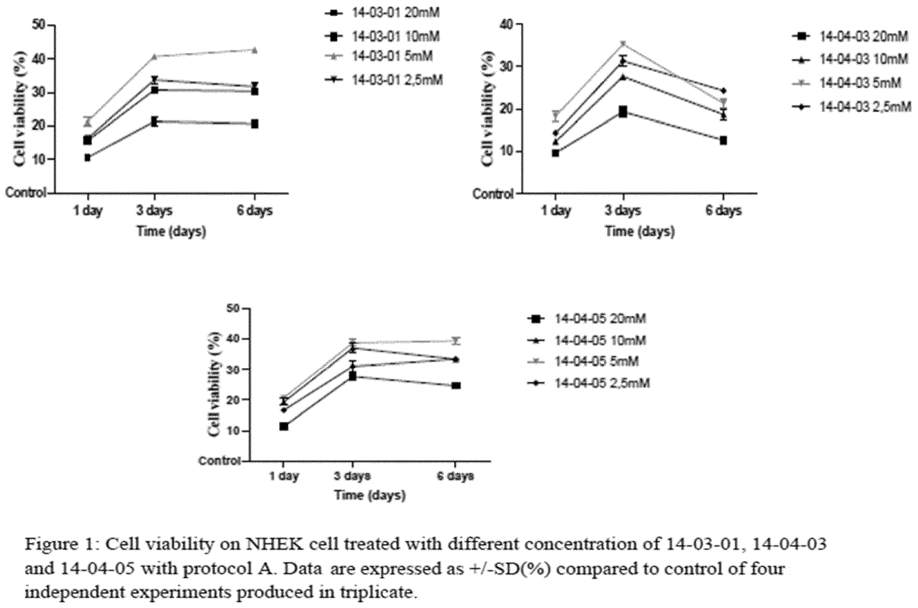

The dose-response study was conducted evaluating the best concentration within the range found in the literature. This test allowed to determine the metabolism of the mitochondrion by analyzing the administration protocols. As shown in Figure 1 the protocol A showed a dose-response effect for all substances with a peak around three days of stimulation; this effect can stabilize only with 14-03-01 and the main effect was observed with 5mM (1:400 dilution).

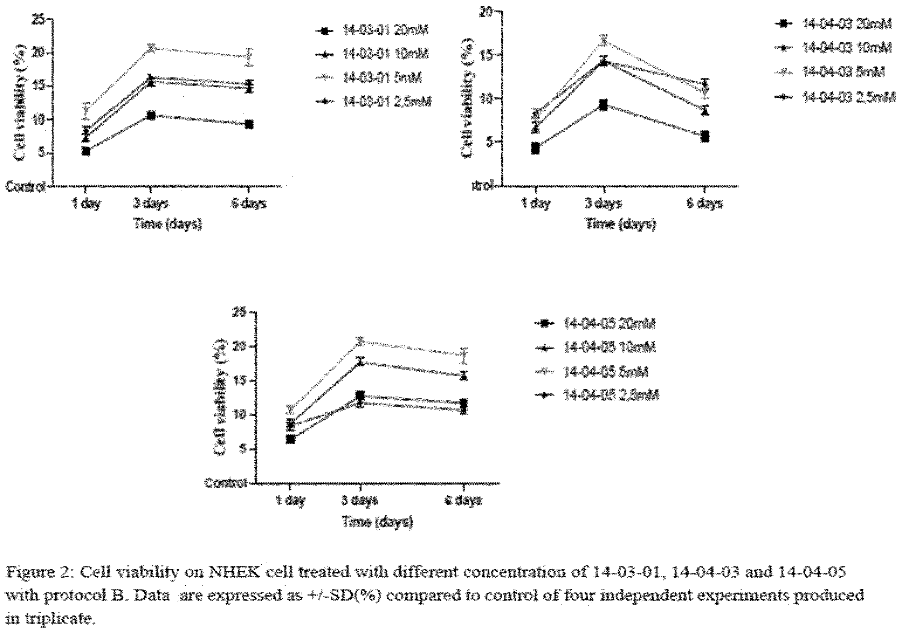

The same tests were also conducted with protocol B (Figure 2) in which the tested composition was administered one treatment/day for 6 days. Also, in this case the best results were obtained by 5mM (p<0.05) for all tested solutions. Considering that the results are "lower" than those observed in protocol A, it is assumed that the daily stimulation is excessive but not toxic. The plateau phase tends to be slightly descending, demonstrating that protocol B is excessive and for this reason protocol B was replaced by protocol C (1 treatment / alternate days carried out max 6 days) in subsequent experiments.

### Proliferation Analysis

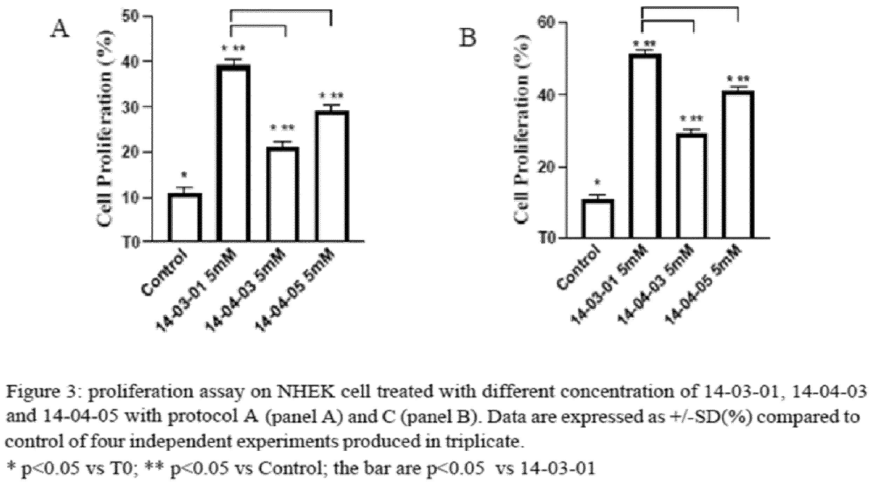

The proliferation assay was conducted using crystal violet on NHEK cells treated with the substances according to protocols A and C. As shown in Figure 3, 14-03-01 significantly increased cell proliferation compared to other compositions (p<0.05), particularly one more time compared to 14-04-03 and about 33% to 14-04-05. In addition, these effects are evident in both protocols, but the main effect was obtained by protocol C (p <0.05 vs A). This is important because by increasing the number of cells it is possible that they can also migrate; in addition, these data suggested an activity of 14-03-01 higher than that of the individual components (p<0.05).

### Effects of 14-03-01, 14-04-03 and 14-04-05 on NHEK Migration

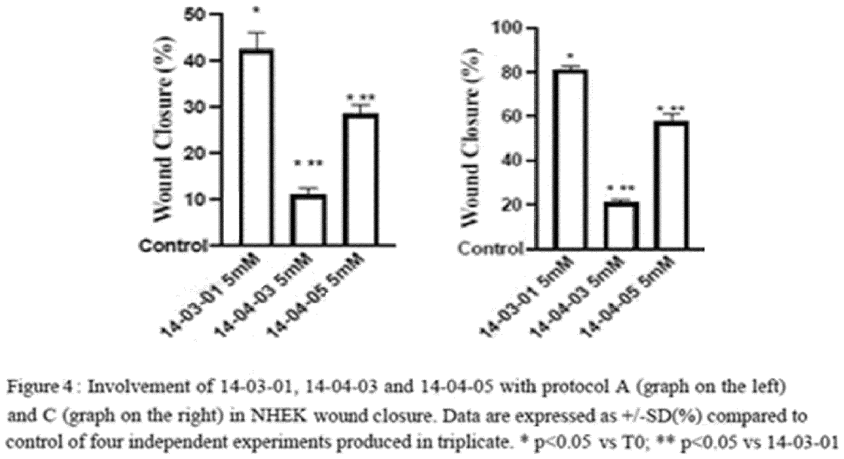

The effect of the different formulations on NHEK migration in wound healing were evaluated (Figure 4). Our results demonstrated an improvement in migration activity (p<0.05) in NHEK cells treated with 14-03-01 (both protocol A and C) compared to control and compared to 14-04-03 (about 3 times more) and 14-04-05 (about 60%). This is a validated method to characterize various factors involved in migration. The data confirm what observed during proliferation analysis.

### Effects of formulations on ROS Production

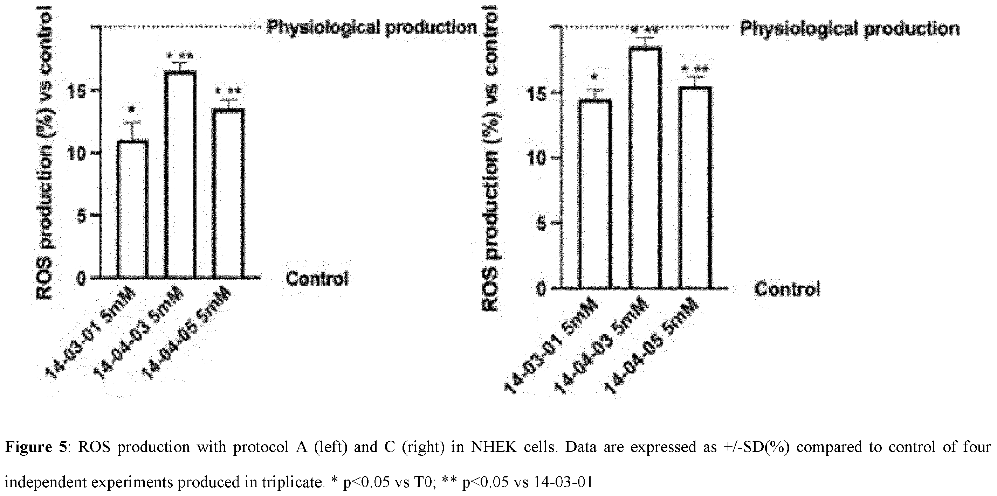

Since conflicting data are reported in literature on the ROS role on healing, further experiments were carried out to explore the role of ROS in this context. As shown in Figure 5, despite the presence of H₂O₂, ROS production does not increase above the physiological share of the tested substances; this is certainly important to ensure the effectiveness observed in previous tests. Obviously, the compound with hydrogen peroxide has a higher ROS production than the other substances in both protocols but 14-03-01 is able to remain below the physiological level of ROS compared to the control and compared to 14-04-03 (about 35%) and compared to 04-14-05 (about 23%), confirming once again the synergistic effect of TCA and hydrogen peroxide to neutralize the negative consequence of the two components administered individually. ROS production is dose dependent since protocol C gives higher results than protocol A but still physiological.

### Methods and results of the in vivo tests

In order to validate the data obtained *in vitro, in vivo* tests were performed using 60 animals (C57BL / 6JOlaHsd mouse) of 8 weeks weighing 20-25g. The method allows to compare different preparations with a specific control by monitoring the wound healing area.

### Evaluation of wound healing area

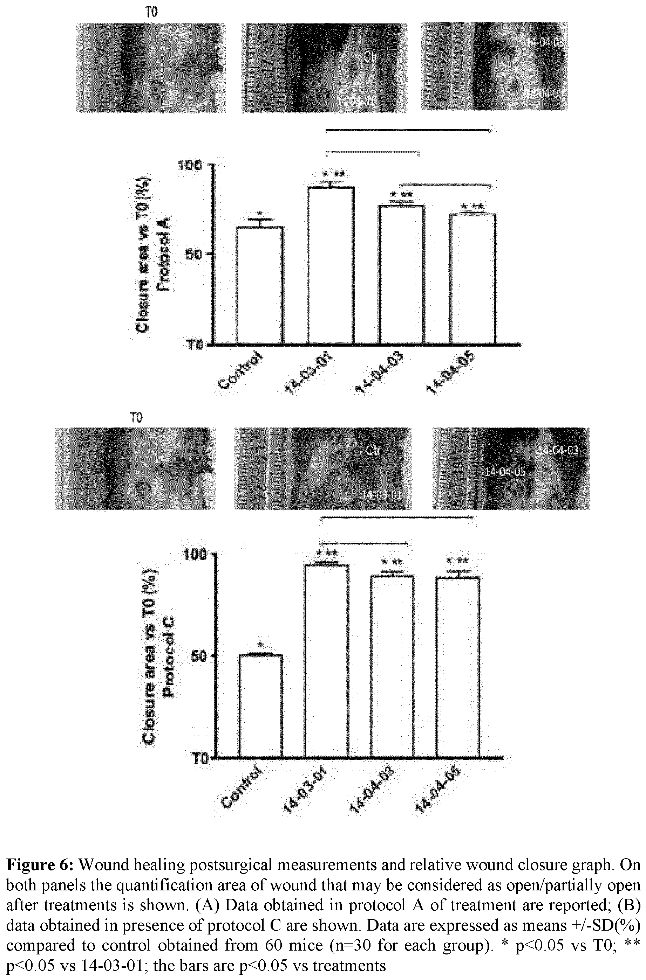

The experimental model of wound healing was performed in male mice treated for 8 days using 14-03-01, 14-04-03 and 14-04-05. As reported in Fig. 6, in protocol A, 14-03-01 induced a greater closure area than control (p < 0.05) and then compared to 14-04-03 and 14-04-05 (14% and 33% respectively). This effect was also statistically significant (p < 0.05) in comparison with untreated wounds. In protocol C the presence of 14-03-01 induced a faster closure in comparison to control (about 98%), 14-04-03 (about 2%) and 14-04-05 (about 4%).

These findings have been confirmed by morphological analysis with E/E and Masson analysis in which the formulation 14-03-01 seems to be a better choice. In all treatments of protocol A, the granulation tissue seemed to be still very active, and the healing was at an early stage. In particular, 14-03-01 showed a quite evident capacity to repair wound and re-epithelization was almost complete. In contrast, protocol C showed that the mice treated with 14-03-01 presented a complete epithelialization of the wound compared to control, to 14-04-03 and to 14-04-05, respectively. Epithelialization appeared without any ulcer, with the presence of inactive granulation tissue, with collagen remodeling and angiogenesis; for this reason, it is possible to envisage a *restitutio ad integrum.*

## Claims

1. Wound healing compositions in form of cream, ointment, liquid or gel comprising:
- a first component selected from trichloroacetic in a concentration by weight ranging from 0.08 to 38%;
- a second component selected from 29-31% aqueous hydrogen peroxide in a concentration by weight ranging from 0.01 to 5% or trichloroperoxyacetic acid wherein:
- the relative molar ratio between trichloroacetic acid and hydrogen peroxide is from 4.34 to 4.74; or
- the relative molecular ratio between trichloroperoxyacetic acid and trichloroacetic acid is from 3.6 to 4.0;
said compositions having a pH value from 2.6 to 3.6

2. Compositions according to claim 1 in form of a cream containing 20-25% w/w water, 5-12% w/w liquid paraffin, 3-7% w/w propylene glycol, 8-12% w/w glycerin, 10-14% w/w of a 28-32% w/w aqueous ammonia solution and 1-2% w/w polysorbate 60.

3. Compositions according to claims 1 or 2 in form of an ointment containing 5-12% w/w water, 0.5-1.5% w/w sodium laurylsulfate, 10-15% w/w propylene glycol, 20-30 w/w stearyl alcohol, 20-30% w/w white petrolatum, 4-6% w/w aqueous ammonia (28-32%) and optionally one or more preservatives in a concentration between 0.02 and 0.05%.

4. Compositions according to claim 3 wherein no preservatives are used.

5. Compositions according to claim 1 in form of a liquid containing 38-99% w/w water, 0.001-0.5% w/w EDTA disodium salt, 0.025-15% w/w glycerol, 0.03-14% w/w aqueous ammonia (28-32%), 0.005-5% of a thickening and texture agent.

6. Compositions according to claim 1 in form of a gel containing 25-35% w/w ethanol, 11-15% w/w propylene glycol, 10-14 w/w of an aqueous ammonia (28-32% w/v), 2-5% w/w diethylene glycol monoethyl ether and 1-4% myristyl alcohol.

7. Compositions according to claim 1 diluted with a 0.9% NaCl aqueous solution.

8. Compositions according to claim 1 wherein trichloroperoxyacetic acid is prepared in situ by addition of hydrogen peroxide to trichloroacetic acid.

9. Compositions of claims 1-8 for use in the treatment of unhealed wounds, ulcers and bedsores, of post-operative complications to tooth extraction including dry socket, dysesthesia, severe infection, pain, swelling, trismus and haemorrhage, of slowly healing wounds, spontaneous ulcerations, sores of various origins and necrotic sores including, pyogenic granulomas, chalazion, epulis, fistulas and abscesses.

## Patentansprüche

1. Wundheilungszusammensetzungen in Form von Creme, Salbe, Flüssigkeit oder Gel, umfassend:
- eine erste Komponente, ausgewählt aus Trichloressigsäure in einer Gewichtskonzentration im Bereich von 0,08 bis 38%;
- eine zweite Komponente, ausgewählt aus 29-31% wässrigem Wasserstoffperoxid in einer Gewichtskonzentration im Bereich von 0,01 bis 5% oder Trichlorperoxyessigsäure, wobei:
- das relative Molverhältnis zwischen Trichloressigsäure und Wasserstoffperoxid von 4,34 bis 4,74 beträgt; oder
- das relative Molverhältnis zwischen Trichlorperoxyessigsäure und Trichloressigsäure von 3,6 bis 4,0 beträgt;
wobei die Zusammensetzungen einen pH-Wert von 2,6 bis 3,6 aufweisen.

2. Zusammensetzungen nach Anspruch 1 in Form einer Creme, enthaltend 20-25% Gew./Gew. Wasser, 5-12% Gew./Gew. flüssiges Paraffin, 3-7% Gew./Gew. Propylenglykol, 8-12% Gew./Gew. Glycerin, 10-14% Gew./Gew. einer 28-32% Gew./Gew. wässrigen Ammoniaklösung und 1-2% Gew./Gew. Polysorbat 60.

3. Zusammensetzungen nach Anspruch 1 oder 2 in Form einer Salbe, enthaltend 5-12% Gew./Gew. Wasser, 0,5-1,5% Gew./Gew. Natriumlaurylsulfat, 10-15% Gew./Gew. Propylenglykol, 20-30% Gew./Gew. Stearylalkohol, 20-30% Gew./Gew. weißes Petrolatum, 4-6% Gew./Gew. wässrigen Ammoniak (28-32%) und gegebenenfalls ein oder mehrere Konservierungsmittel in einer Konzentration zwischen 0,02 und 0,05%.

4. Zusammensetzungen nach Anspruch 3, wobei keine Konservierungsmittel verwendet werden.

5. Zusammensetzungen nach Anspruch 1 in Form einer Flüssigkeit, enthaltend 38-99% Gew./Gew. Wasser, 0,001-0,5% Gew./Gew. EDTA-Dinatriumsalz, 0,025-15% Gew./Gew. Glycerin, 0,03-14% Gew./Gew. wässrigen Ammoniak (28-32%), 0,005-5% eines Verdickungs- und Texturmittels.

6. Zusammensetzungen nach Anspruch 1 in Form eines Gels, enthaltend 25-35% Gew./Gew. Ethanol, 11-15% Gew./Gew. Propylenglykol, 10-14 Gew./Gew. eines wässrigen Ammoniaks (28-32% Gew./Vol.), 2-5% Gew./Gew. Diethylenglykolmonoethylether und 1-4% Myristylalkohol.

7. Zusammensetzungen nach Anspruch 1, verdünnt mit einer 0,9%igen wässrigen NaCl-Lösung.

8. Zusammensetzungen nach Anspruch 1, wobei Trichlorperoxyessigsäure in situ durch Zugabe von Wasserstoffperoxid zu Trichloressigsäure hergestellt wird.

9. Zusammensetzungen nach den Ansprüchen 1-8 zur Verwendung bei der Behandlung von ungeheilten Wunden, Geschwüren und Bettwunden, von postoperativen Komplikationen bei der Zahnextraktion, einschließlich trockener Alveole, Dysästhesie, schwerer Infektion, Schmerzen, Schwellungen, Trismus und Blutungen, von langsam heilenden Wunden, spontanen Geschwüren, Wunden verschiedenen Ursprungs und nekrotischen Wunden, einschließlich pyogener Granulome, Chalazion, Epulis, Fisteln und Abszessen.

## Revendications

1. Compositions cicatrisantes sous forme de crème, de pommade, de liquide ou de gel comprenant :
- un premier constituant choisi parmi l'acide trichloroacétique à une concentration en poids allant de 0,08 à 38 % ;
- un second constituant choisi parmi le peroxyde d'hydrogène aqueux à 29-31% à une concentration en poids allant de 0,01 à 5 % ou l'acide trichloroperoxyacétique, dans lesquelles :
- le rapport molaire relatif entre l'acide trichloroacétique et le peroxyde d'hydrogène est de 4,34 à 4,74 ; ou
- le rapport moléculaire relatif entre l'acide trichloroperoxyacétique et l'acide trichloroacétique est de 3,6 à 4,0 ;
lesdites compositions ayant une valeur de pH de 2,6 à 3,6.

2. Compositions selon la revendication 1, sous forme de crème contenant 20 à 25 % p/p d'eau, 5 à 12 % p/p de paraffine liquide, 3 à 7 % p/p de propylène glycol, 8 à 12 % p/p de glycérine, 10 à 14 % p/p d'une solution aqueuse d'ammoniac à 28-32 % p/p et 1 à 2 % p/p de polysorbate 60.

3. Compositions selon les revendications 1 ou 2, sous forme de pommade contenant 5 à 12 % p/p d'eau, 0,5 à 1,5 % p/p de laurylsulfate de sodium, 10 à 15 % p/p de propylène glycol, 20 à 30 % p/p d'alcool stéarylique, 20 à 30 % p/p de vaseline blanche, 4 à 6 % p/p d'ammoniaque (28 à 32 %) et éventuellement un ou plusieurs conservateurs à une concentration comprise entre 0,02 et 0,05 %.

4. Compositions selon la revendication 3, dans lesquelles aucun conservateur n'est utilisé.

5. Compositions selon la revendication 1, sous forme liquide contenant 38 à 99 % p/p d'eau, 0,001 à 0,5 % p/p de sel disodique d'EDTA, 0,025 à 15 % p/p de glycérol, 0,03 à 14 % p/p d'ammoniaque (28 à 32 %), et 0,005 à 5 % d'un agent épaississant et texturant.

6. Compositions selon la revendication 1, sous forme de gel contenant 25 à 35 % p/p d'éthanol, 11 à 15 % p/p de propylène glycol, 10 à 14 % p/p d'ammoniaque (28 à 32 % p/v), 2 à 5 % p/p d'éther monoéthylique de diéthylène glycol et 1 à 4 % d'alcool myristylique.

7. Compositions selon la revendication 1, diluées avec une solution aqueuse de NaCl à 0,9 %.

8. Compositions selon la revendication 1, dans lesquelles l'acide trichloroperoxyacétique est préparé in situ par ajout de peroxyde d'hydrogène à l'acide trichloroacétique.

9. Compositions selon les revendications 1 à 8 pour une utilisation dans le traitement des plaies non cicatrisées, des ulcères et des escarres, des complications postopératoires à l'extraction dentaire, y compris l'alvéolite, la dysesthésie, l'infection grave, la douleur, le gonflement, le trismus et l'hémorragie, des plaies à cicatrisation lente, des ulcérations spontanées, des plaies d'origines diverses et des plaies nécrotiques, y compris les granulomes pyogènes, le chalazion, l'épulis, les fistules et les abcès.
